# EUROPEAN PATENT APPLICATION

(11) **EP 2 698 151 A1**
(43) Date of publication of application: **19.02.2014**
(21) Application number: 13180634.1
(22) Date of filing: 16.08.2013
(51) Int. Cl.: A61K 9/20, A61K 31/00

(54) **Orally Disintegrating Formulation of Paliperidone**

(30) Priority: 17.08.2012 TR 201209598
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

The present invention is related to an orally disintegrating composition comprising paliperidone and one or more pharmaceutically acceptable excipient.

## Description

### Technical Aspect

The present invention is related to an orally disintegrating composition comprising paliperidone and one or more pharmaceutically acceptable excipient.

### Background of the Present Invention

Paliperidone corresponding to the compound 4H-Pyrido[1,2-a]pyrimidin-4-one,3-[2-[4-(6-fluoro-1,2-benzisoxazol-3-yl)-1- piperidinyl]ethyl]-6,7,8,9-tetrahydro-9- hydroxy-2-methyl is an antipsychotic agent which is approved for the treatment of schizophrenia including acute treatment and recurrence prevention. Paliperidone, first described in US5254556A, is available as Invega® Extended Release Tablet for oral administration.

In the field of pharmacy, compliance and convenience with medication is a major problem, particularly for psychotic patients or patients who have difficulty of swallowing solid medications. That is one of the reasons why orally disintegrating compositions are needed. Orally disintegrating compositions are becoming an increasingly important issue in the area of better patient compliance and convenience comparative to the conventional solid dosage forms for oral administration such as capsules and tablets.

Many solid oral dosage forms of paliperidone are disclosed in prior art. However, none of them includes orally disintegrating forms. EP2079446 is about a tablet comprising a first layer of paliperidone with at least one release controlling polymer and an outer layer coating partially this first layer. Furthermore, EP2326312 discloses solid pharmaceutical composition comprising at least one solid matrix particle of paliperidone which is a monolithic matrix system. Furthermore, the patent US5254556A discloses oral drops, oral solution, capsules, film-coated tablets, injectable solution and suppositories.

In addition to the advantage of swallowing facility, orally disintegrating compositions can be administrated in situations where no water is available to dissolve a tablet. Furthermore, for many medicaments, the act of swallowing the medicament often requires fluids that increase gastric volume and the likelihood of nausea and vomiting. Perhaps the biggest advantage of orally disintegrating compositions is that the solid dosage form dissolves or disintegrates quickly in the oral cavity, resulting in a solution or suspension without the need for the administration of fluid. Accordingly, the patient can administer the dosage form as soon as symptoms are felt. Thus, the orally disintegrating compositions is one of the advantageous methods to deliver the drugs such as comprising antipsychotic agents to such patients and provide a better patient compliance with recommended pharmaceutical therapies.

Additionally oral administration of the drugs is difficult in patients having concomitant vomiting, nausea or diarrhoea. The orally disintegrating dosage form is one of the advantageous methods to deliver the drugs to such patients. By administering the orally disintegrating dosage forms, faster absorption of the drug occurs through buccal mucosa and it may reduce the first pass metabolism leading to better efficacy of the drug. This dosage form enhances the clinical effects of some drugs by leading to an increase in bioavailability and a reduction in side effects because of avoidance of firstpass liver metabolism.

In addition to these problems in prior art, some psychiatric patient populations exhibit "cheeking" behavior (i.e., holding the oral dosage form in the cheek) to avoid swallowing the medication. Accordingly, orally disintegrating compositions would be desirable to improve patient compliance, particularly among elderly patients, because orally disintegrating compositions are easier to swallow and prevent "cheeking".

In prior art, there are also several patents which disclose orally disintegrating compositions and manufacturing technologies thereof; but none of them includes paliperidone. These technologies developed for manufacturing orally disintegrating compositions can be exemplified as freeze drying, molding and compaction technologies. Despite of these technologies, there are still difficulties to develop orally disintegrating compositions because of several different reasons. A satisfied orally disintegrating dosage form needs to meet a number of requirements. To allow a quick absorption of a therapeutic agent, a short disintegration or dissolution time in the oral cavity is needed. Moreover, a premature release in the mouth could also lead to problems due to the often unpleasant taste of the active ingredient. Besides, these compositions should be very porous and should not be very hard. These porous compositions tend to be very sensitive to humidity. As a consequence, they may have some stability problems.

To fulfill all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully.

Additionally, precautions have to be taken at the preparation, packaging, handling and storing of the finished dosage forms of orally disintegrating compositions since they tend to be both hygroscopic and friable.

Thus, a need rises and the present invention discloses formulations for orally disintegrating compositions of paliperidone or pharmaceutically acceptable salts thereof which overcome above described problems and which further provide the advantageous property of allowing the active medicament to disintegrate or dissolve rapidly in the oral cavity which have a pleasant mouth feel and good mechanical strength, enough to be processed in high speed tableting machines and shipped in low cost packages.

### Object of the Invention

The main object of the present invention is to obtain an orally disintegrating novel composition with antipsychotic activity, which is capable of providing an increased patient compliance and convenience and an easy administration.

Another object of the present invention is to provide orally disintegrating composition which avoids the afore-mentioned disadvantages of the orally disintegrating compositions of the prior art and have advantages over them.

Yet another object of the present invention is to provide orally disintegrating composition allowing the active agent to disintegrate and dissolve rapidly in the oral cavity by preventing unpleasant taste that may arise in mouth and esophagus during its passage through it.

A further object of the present invention is to provide orally disintegrating composition which have good mechanical strength (such as adequate hardness and low friability) enough to be processed in high speed tableting machines and shipped in low cost packages.

Another object of the present invention is to provide bioavailable and stable orally disintegrating composition of paliperidone.

Another object of the present invention is to provide a simple, cost-effective and time saving process for the preparation of orally disintegrating composition of paliperidone.

### Detailed Description of the Invention

The present invention is directed to novel orally disintegrating composition comprising paliperidone and one or more pharmaceutically acceptable excipient.

The term "paliperidone" as used herein refers to paliperidone as well as pharmaceutically acceptable salts, enantiomers, hydrates, solvates, metabolites, prodrugs and mixture thereof. The term also includes all polymorphic forms, whether crystalline or amorphous.

According to an embodiment of the present invention, said orally disintegrating composition contains paliperidone in an amount of 0.1% to 25% by weight of total formulation, preferably in an amount of 1 % to 20%. In a further embodiment of the present invention, the amount of paliperidone is 0.01 mg to 20 mg and preferably 0.1 mg to 10 mg.

The orally disintegrating compositions of this invention further comprising one or more pharmaceutically acceptable excipient selected from the group comprising disintegrants, diluents, dispersing agents, binders, lubricants, glidants, stabilizers, preservatives, sweeteners, flavorings and coloring agents.

According to a preferred embodiment of the present invention, the orally disintegrating composition comprise one or more disintegrant as a pharmaceutically acceptable excipient.

Suitable disintegrants may include but not limited to cross-linked polyvinil pyrrolidone (crospovidone), cross-linked carboxymethyl cellulose (croscarmellose sodium), low-substituted hydroxypropyl cellulose, pregelatinized starch, sodium carboxymethyl cellulose, calcium carboxymethyl cellulose, sodium alginate, corn starch, sodium starch glycolate and mixtures thereof. The preferred disintegrant for the composition of the present invention is crospovidone. Disintegrant content is from 0.5% to 30% by weight of the orally disintegrating composition, preferably is present from 1% to 20% by weight of total composition.

It has unexpectedly been found that the specific combination of paliperidone with disintegrants provides an orally disintegrating composition which avoids the afore-mentioned disadvantages of the orally disintegrating compositions of the prior art. In a preferred embodiment of the present invention, the ratio of paliperidone to disintegrant is in the range of between 1:10 and 10:1 (w/w) and preferably is between 1:5 and 5:1 (w/w). It has also been found that the combination of paliperidone with the disintegrant, particularly crospovidone, in the ratio suitable to the present invention, has a synergistic effect over the disintegration profile. Preferably this ratio of the combination of paliperidone with the disintegrant, particularly crospovidone, makes the composition having improved compressibility, reduced friability and at the same time, reduced disintegration time.

In one embodiment the orally disintegrating composition of paliperidone starts to disintegrate when in contact with the moisture of the buccal cavity before being swallowed. In a further embodiment, the composition disintegrates in oral cavity in less than 90 seconds, preferably in less than 60 seconds, more preferably in less than 30 seconds. Disintegration time is determined by a method comprising the steps of placing individual tablets withing a USP basket-rack assembly and of lowering the basket into purified water maintained at 37°C and at a constant frequency rate of between 25 and 35 cycles per minute.

In a preferred embodiment of the present invention, the hardness of the orally disintegrating composition can range from 10 to 180 N and preferably 15 to 120 N. Hardness of the orally disintegrating composition is tested according to standardized methods and equipment provided in European Pharmacopeia.

Suitable diluents may include but not limited to microcrystalline cellulose, mannitol, spray-dried mannitol, lactose, starch, dextrose, sucrose, fructose, maltose, sorbitol, xylitol, inositol, kaolin, sodium carbonate, sodium bicarbonate, calcium carbonate and mixtures thereof; preferably the diluent is microcrystalline cellulose, mannitol, spray-dried mannitol or the mixtures thereof and more preferably the diluent is mannitol. Diluent content is from 1% to 80% by weight of the orally disintegrating composition, preferably is present from 10% to 60% by weight of total composition.

In a preferred embodiment of the present invention, the ratio of disintegrant to diluent is in the range of 1:0.1 and 60:1 (w/w). Said amount makes the disintegrant and diluent possible to significantly improve mechanical strength of the orally disintegrating tablet such as compressibility and hardness, also reduce friability. In a further embodiment of the present invention, disintegrant and diluent used to form the ratio of disintegrant to diluent are crospovidone and mannitol. Crospovidone is an insoluble polymer with high swelling capacity. This capacity helps crospovidone to dissolve easily and quickly in a little amount of water or saliva and makes its disintegrating rate much faster than other related excipients. Furthermore, mannitol is easily and quickly soluble in water or saliva. Thus, a formulation comprising crospovidone with mannitol has improved disintegration profile. As well, mannitol is highly compressible and it has optimum fluidity for direct compression processes. It has good dilution capacity due to the size and form of the particle, which makes it possible to accept large amounts of active ingredients that are not easily compressed. It is chemically very stable and non-hygroscopic.

In a preferred embodiment of the present invention, the orally disintegrating composition of paliperidone comprises;
a. Paliperidone as an active agent,
b. Crospovidone,
c. Mannitol,
wherein the ratio of paliperidone to crospovidone is in the range of between 1:10 and 10:1 (w/w) and the ratio of crospovidone to mannitol is in the range of 1:0.1 and 60:1 (w/w).

Suitable dispersing agents may include but not limited to calcium silicate, magnesium aluminum silicate and mixtures thereof.

Suitable binders may include but not limited to polyvinylpyrrolidone, polyethylene glycol, polyvinyl alcohol, starch, pregelatinized starch, glucose, sucrose, sodium alginate, cellulose derivatives such as hydroxypropyl methyl cellulose, hydroxypropyl cellulose, carboxy methyl cellulose, methyl cellulose, gelatin, carrageenan, guar gum, carbomer, methacrylate polymers, xanthan gum and mixtures thereof. Binder content is from 1% to 20%, preferably from 2% to 10% by weight of total composition.

Suitable lubricants may include but not limited to magnesium strearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, sodium stearyl fumarate, sodium lauryl sulphate and mixtures thereof; preferably the lubricant is magnesium strearate and sodium lauryl sulfate. Lubricant content is from 0.01% to 10% by weight of total composition.

Suitable glidants may include but not limited to colloidal silicon dioxide, talc, aluminium silicate and mixtures thereof; preferably the glidant is colloidal silicon dioxide. Glidant content is from 0.02% to 10% by weight of total composition.

Suitable stabilizers may include but not limited to citric acid, fumaric acid, tartaric acid, sodium dihydrogen phosphate, calcium carbonate, magnesium carbonate, arginine, lysine, meglamine and the mixtures thereof.

Suitable preservatives may comprise but not limited to methyl paraben and propyl paraben and their salts (such as sodium, potassium), sodium benzoate, citric acid, benzoic acid, butylated hydroxytoluene and butylated hydroxyanisole and mixtures thereof. In one aspect, the preservative content may present in an amount of about from 0.01% to 5%, preferably about from 0.5% to 2% by weight of total composition.

Suitable sweeteners may include but not limited to aspartame, potassium acesulfame, sodium saccharinate, neohesperidine dihydrochalcone, sucralose, saccharin, sugars such as sucrose, glucose, lactose, fructose and sugar alcohols such as mannitol, sorbitol, xylitol, erythritol and mixtures thereof; preferably the sweetener is aspartame, sucralose and/or saccharin. Sweetener content is from 0.01% to 5%, preferably from 0.02% to 3.0% by weight of total composition.

Suitable flavorings may include but not limited to menthol, peppermint, cinnamon, chocolate, vanillin and fruit essences such as cherry, orange, strawberry, grape, black currant, raspberry, banana, red fruits, wild berries etc. and mixtures thereof; preferably the flavoring agent is menthol and/or fruit essences. Flavoring agent content is from 0.01% to 5%, preferably from 0.1 % to 3% by weight of total composition.

Suitable coloring agents may include but not limited to ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow and mixtures thereof.

In this present invention, to minimize the disintegration time and maximise the mechanical resistance of the tablets of this invention, this orally disintegrating tablet composition has been designed, made up of the following:
(a) 0.1 to 25 % by weight of paliperidone,
(b) 0.5 to 30 % by weight of disintegrant,
(c) 1 to 80 % by weight of diluent,
(d) 1 to 30 % by weight of binder,
(e) 0.02 to 10 % by weight of glidant,
(f) 0.01 to 10 % by weight of lubricant,
(g) 0.01 to 5 % by weight of sweetener,
(h) 0.01 to 5 % by weight of flavoring agent.

The orally disintegrating compositions of this invention include tablets, sachets, minitablets, multilayer and multicoated tablets, pellets or powders which can be formulated in accordance with methods that are standard in the art. It is preferably in the form of tablets.

In a further aspect, the present invention shows that it is possible to have a significant influence on the disintegration rate of the tablet by modifying the dimensions and shape of the tablet. In general, as the tablet becomes thinner and have higher porosity, the orally disintegrating composition will be weakened faster when it contacts with saliva, because the disintegration process is produced after wetting all the surface of the tablet via capillary action. Also, any shape which maximizes the contact surface with the saliva may produce a significant reduction in disintegration time.

The preferred shape of the orally disintegrating tablet composition of this invention may have a shape of a disk, circle, round, sphere, donut, bar, polygon, ellipse and the like.

In one embodiment, the orally disintegrating compositions of the present invention may be prepared by conventional technology well known to those skilled in the art such as direct compression, dry granulation, wet granulation and the like. During direct compression, active agent and excipients are mixed, sieved and compressed into dosage forms. During wet granulation, the ingredients are mixed and granulated with a granulation liquid. The granulation process provides agglomerates with a desired homogeneity. The mixture is sieved and optionally mixed with additional excipients. Finally, it is compressed into dosage forms.

A preferred process for preparing the orally disintegrating compositions of the invention comprises the following steps:
a) mixing paliperidone, with other excipients such as disintegrants, diluents and binders;
b) blending the mixture with a glidant and lubricant;
c) optionally adding other excipients such as sweetners, flavouring agents, preservatives, colouring agents;
d) compressing the blended mixture to form tablets.

This invention is further defined by reference to the following examples. Although the examples are not intended to limit the scope of the present invention, it should be considered in the light of the description detailed above.

### Examples:

### Example 1

| **Ingredients:** | **Amount: (*by weight of the total composition*)** |
|---|---|
| Paliperidone | 0.1 to 25% |
| Crospovidone | 0.5 to 30% |
| Mannitol | 1 to 80% |
| Croscarmellose sodium | 0.5 to 30 % |
| Low-substitued hydroxypropyl cellulose | 1 to 30 % |
| Aspartame | 0.02 to 5 % |
| Flavoring agent | 0.01 to 5% |
| Talc | 0.05 to 10% |
| Colloidal silicon dioxide | 0.02 to 10% |
| Magnesium stearat | 0.02 to 10% |

### Example 2

| **Ingredients:** | **Amount: (*by weight of the total composition*)** |
|---|---|
| Paliperidone | 0.1 to 25% |
| Crospovidone | 0.5 to 30% |
| Spray dried mannitol | 1 to 80% |
| Croscarmellose sodium | 0.5 to 30 % |
| Microcrystalline cellulose | 1 to 80 % |
| Sodium saccharin | 0.02 to 2 % |
| Flavoring agent | 0.01 to 5% |
| Colloidal silicon dioxide | 0.02 to 10% |
| Magnesium stearat and sodium lauryl sulfate | 0.02 to 10% |

### Example 3

| **Ingredients:** | **Amount: (*by weight of the total composition*)** |
|---|---|
| Paliperidone | 0.1 to 25% |
| Crospovidone | 1 to 30 % |
| mannitol | 1 to 80% |
| Calcium silicate | 1 to 90 % |
| Sucralose | 1 to 2 % |
| Sodium saccharin | 0.02 to 2 % |
| Flavoring agent | 0.1 to 5% |

The formulations of these examples are manufactured according to the process described above in the description.

## Claims

1. An orally disintegrating composition comprising paliperidone, a disintegrant and one or more pharmaceutically acceptable excipients, wherein the composition disintegrates in oral cavity in less than 90 seconds.

2. The orally disintegrating composition according to claim 1, paliperidone is present in an amount of 0.10 to 25% by weight of total composition, preferably it is 1 to 20% by weight of total composition.

3. The orally disintegrating composition according to claim 1, disintegrant is present in an amount of 0.5 to 30% by weight of the total composition.

4. The orally disintegrating composition according to claim 3, disintegrant is present in an amount of 1 to 20% by weight of the total composition.

5. The orally disintegrating composition according to any preceding claims, the ratio of paliperidone to disintegrant is in the range of between 1:10 and 10:1 (w/w).

6. The orally disintegrating composition according to any preceding claims, disintegrants are selected from the group comprising cross-linked polyvinil pyrrolidone (crospovidone), cross-linked carboxymethyl cellulose (croscarmellose sodium), low-substituted hydroxypropyl cellulose, pregelatinized starch, sodium carboxymethyl cellulose, calcium carboxymethyl cellulose, sodium alginate, corn starch, sodium starch glycolate and mixtures thereof.

7. The orally disintegrating composition according to claim 6, disintegrant is crospovidone.

8. The orally disintegrating composition according to claim 1, the one or more pharmaceutically acceptable excipients are selected from the group comprising diluents, dispersing agents, binders, lubricants, glidants, surfactants, stabilizers, preservatives, sweeteners, flavorings and coloring agents.

9. The orally disintegrating composition according to claim 8, diluents are selected from the group comprising microcrystalline cellulose, mannitol, spray-dried mannitol, lactose, starch, dextrose, sucrose, fructose, maltose, sorbitol, xylitol, inositol, kaolin, sodium carbonate, sodium bicarbonate, calcium carbonate and mixtures thereof.

10. The orally disintegrating composition according to claim 9, diluents are selected from the group comprising microcrystalline cellulose, mannitol, spray dried mannitol and the mixtures thereof.

11. The orally disintegrating composition according to claim 10, diluent is mannitol.

12. The orally disintegrating composition according to claim 9 to 11, diluent is present in an amount of 1 to 80% by weight of the total composition.

13. The orally disintegrating composition according to claim 12, diluent is present in an amount of 10 to 60% by weight of the total composition.

14. The orally disintegrating composition according to claim 1, comprising;
a) paliperidone as an active agent,
b) crospovidone as a disintegrant,
c) mannitol as a diluent,
wherein the composition disintegrates in oral cavity in less than 90 seconds.

15. The orally disintegrating composition according to claim 14, the ratio of crospovidone to mannitol is in the range of 1:0.1 and 60:1 (w/w).

16. The orally disintegrating composition according to claim 14, the ratio of paliperidone to crospovidone is in the range of between 1:10 and 10:1 (w/w).

17. The orally disintegrating composition according to any preceding claims, comprising;
0.1 to 25 % by weight of paliperidone,
0.5 to 30 % by weight of disintegrant,
1 to 80 % by weight of diluent,
1 to 30 % by weight of binder,
0.02 to 10 % by weight of glidant,
0.01 to 10 % by weight of lubricant,
0.01 to 5 % by weight of sweetener,
0.01 to 5 % by weight of flavoring agent.

18. A process for preparing orally disintegrating compositions according to any preceding claim comprising;
mixing paliperidone, with other excipients such as disintegrants, diluents and binders;
blending the mixture with a glidant and lubricant;
optionally adding other excipients such as sweetners, flavouring agents, preservatives, colouring agents;
compressing the blended mixture to form tablets.
